# EUROPÄISCHE PATENTSCHRIFT

(11) **EP 1 000 610 B1**
(45) Veröffentlichungstag und Bekanntmachung des Hinweises auf die Patenterteilung: **07.04.2004**
(21) Anmeldenummer: 99121703.5
(22) Anmeldetag: 02.11.1999
(51) Int. Cl.: A61K 7/11

(54) **Polymere für kosmetische Formulierungen**
Polymers for cosmetic formulations
Polymères pour formulations cosmétiques

(30) Priorität: 02.11.1998 DE 19850363
(43) Veröffentlichungstag der Anmeldung: 17.05.2000
(73) Patentinhaber: BASF AKTIENGESELLSCHAFT, 67056 Ludwigshafen (DE)
(72) Erfinder: Witteler, Helmut, Dr., 67259 Beindersheim (DE); Dieing, Reinhold, Dr., 67105 Schifferstadt (DE); Sanner, Axel, Dr., 67227 Frankenthal (DE); Engesser, Jacqueline, 67071 Ludwigshafen (DE); Dausch, Wilma M., Dr., 67117 Limburgerhof (DE)

(56) Entgegenhaltungen:
- US-A- 3 645 965

## Beschreibung

Die Erfindung betrifft die Verwendung von Polymeren, an die Gruppen der Formel I gebunden sind, 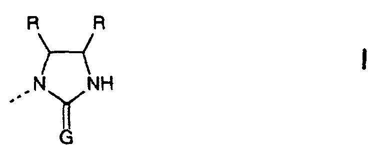 wobei
- R: für H steht oder beide R für die Gruppe -NH-C(O)-NH- und
- G: für O oder NH steht,
in Kosmetika und als Haarfestigerpolymere.

In haarkosmetischen Formulierungen werden vielfach Polymere eingesetzt. Anforderungen, die an haarkosmetische Mittel gestellt werden, sind unter anderem bei B. Hoffbauer, R. Brott, Aerosol and Spray Report 36 (10), 9-15 (1997) und bei Dale H. Johnson (Hrsg.), "Hair and Hair Care", Marcel Dekker Inc., New York 1997, S. 105-165, beschrieben.

Aufgrund der ständig wachsenden Forderung nach umweltverträglicheren Erzeugnissen soll der Gehalt der flüchtigen organischen Verbindungen (engl.: volatile organic compounds-VOC) in kosmetischen Zubereitungen, wie beispielsweise Haarsprays, Haargele oder Schaumfestiger, möglichst niedrig gehalten oder gesenkt werden. Bei Verwendung herkömmlicher Polymere bereitet dies jedoch Schwierigkeiten, da mit den Wassergehalten von Haarsprayformulierungen ihre Viskosität steigt und eine gleichmäßige Aufbringung des Polymers auf das Haar nicht mehr gewährleistet ist. Die anschließende Verfilmung führt zu unbefriedigenden Resultaten hinsichtlich Glanz, Griff und Haftung. Es ist versucht worden, diesem Problem durch Verwendung von Polymeren auf Basis von Polyamiden (DE 44 28 003) oder Polyestern und Polyesteramiden (US 5 158 762) zu begegnen. Diese Polymere zeigen jedoch entweder Nachteile in der Auswaschbarkeit mit Wasser oder es bestehen Defizite bezüglich der Löslichkeit in marktüblichen Treibgas-Lösungsmittel-Gemischen.

In DE 39 02 067 wird die Verwendung wäßriger Kunststoffdispersionen, die aus Polymerisaten, in denen auch ein Ureidomethacrylat als Monomerbaustein zur Anwendung kommt, zur Imprägnierung von Polyestergewebe beschrieben. Als Vorteil wird die Verminderung der Formaldehyd-Freisetzung angegeben. Die Verwendung von Ureidomethacrylat als Copolymer verbessert die Haftung des Polymers auf der Faser.

In DE 39 02 555 wird die Verwendung wäßriger Kunststoffdispersionen, die aus Polymerisaten, in denen auch ein Ureidomethacrylat als Monomerbaustein zur Anwendung kommt, zum Anstrich von Kunststoffoberflächen beschrieben. Als Vorteil wird die verbesserte Adhäsion auf Oberflächen von Hart-PVC, Polycarbonat, ABS, Polystyrol und PPO angegeben.

In AU 8425444 werden Ureidomethacrylat-haltige Vinylacetat-Copolymere beschrieben, die als Binder für Textilvliese verwendbar sind. Die Verwendung von Ureidomethacrylat als Copolymer verbessert die Haftung des Polymers auf der Faser.

Die US 3 645 965 offenbart Polymere, die durch Zugabe eines Imidazolidins stabilisiert werden.

In WO 97/45468 wird über acetacetoxy-modifizierte Polymere mit Tensidwirkung berichtet. Diese sind insbesondere als Beschichtungsmaterialien geeignet. Ihr Einsatz in Shampoos wird auch erwähnt. Die genannten Polymere können neben Acetacetoxygruppen unter anderem auch Ureidomethacrylat enthalten.

Der Erfindung liegt die Aufgabe zugrunde, die Zusammensetzung von Polymeren, die in haarkosmetischen Anwendungen üblicherweise eingesetzt werden oder im Prinzip eingesetzt werden könnten, so zu variieren, daß sich gleichzeitig ihre Verarbeitungs- oder Applikationseigenschaften und ihre Gebrauchseigenschaften verbessern, ohne daß bestimmte Eigenschaften wie Tensidwirkung zwangsweise auftreten. Dies soll insbesondere hinsichtlich Festigung, Auswaschbarkeit und Viskosität während und nach der Applikation gelten. Weitere zu optimierende Eigenschaften ergeben sich aus der jeweiligen Anwendung. So sind bei der Anwendung in Haarsprays konkret folgende Eigenschaften von Interesse: Viskosität, Filmbildung, Festigungswirkung, Klebefreiheit (auch bei hoher Luftfeuchtigkeit), Auskämmbarkeit, Auswaschbarkeit, Griff, Glanz, Haftung, antistatische Wirkung und Löslichkeit in Wasser, Alkohol, Dimethylether, Propan und Butan sowie in Mischungen daraus. Diese Eigenschaften von Haarfestigerpolymeren sowie die Charakterisierung von Polymeren hinsichtlich dieser Eigenschaften sind dem Fachmann bekannt. Ferner sind die Herstellkosten und die Wirkung pro eingesetzter Menge des Polymers von Bedeutung.

Diese Aufgabe wird durch die Verwendung von Polymeren, an die Gruppen der Formel I gebunden sind, 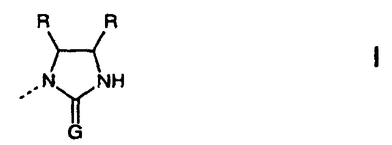 wobei
- R: für H steht oder beide R für die Gruppe -NH-C(O)-NH- und
- G: für O oder NH steht,
als Haarfestigerpolymere,
gelöst.

Gefunden wurde, daß die Verwendung der Polymere P, die eine Gruppe I (Derivate von Ethylenharnstoff) enthalten, in haarkosmetischen Formulierungen von Vorteil ist. Der Vorteil der erfindungsgemäßen Verwendung der Polymere P in haarkosmetischen Formulierungen ergibt sich insbesondere aus einer verbesserten Auswaschbarkeit bei herabgesetzter Lösungsviskosität und unveränderter Festigungswirkung.

Besonders geeignet für die erfindungsgemäße Verwendung sind Polymere P, die unter Einsatz von Monomeren der Formel II hergestellt werden oder Wiederholungseinheiten der Formel III enthalten, 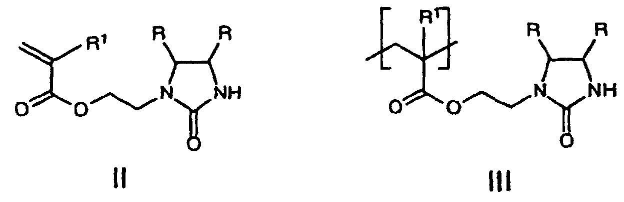
- R¹: steht für H oder CH₃ und
- R: R steht für H oder beide R für die Gruppe -NH-C(O)-NH-.

Überraschenderweise wurde gefunden, daß die Polymere P, obwohl Monomere der Formel II normalerweise in der Polymersynthese als Mittel zur Erhöhung der Adhäsion des Polymers eingesetzt wird, aus menschlichem Haar gut auswaschbar sind. Während mit Ureidomethacrylat-haltigen Bindern für Textilien, wie sie dem Stand der Technik entsprechen, Beschichtungen erhalten werden, deren Auswaschbarkeit durch Ureidomethacrylat verringert ist, zeichnen sich die Polymere P für den erfindungsgemäßen Anwendungszweck, beispielsweise die Verwendung als Haarspray, durch gute Auswaschbarkeit aus. Ferner wird bei der Anwendung in Haarsprays gute Festigungswirkung, guter Griff und gute Kämmbarkeit gefunden, die handelsüblichen Polymeren, beispielsweise Luvimer™ 100 P von der Fa. BASF Aktiengesellschaft, gleichwertig sind. Auch die Lösungsviskosität ist, bei ansonsten gleichen oder verbesserten Gebrauchseigenschaften, niedriger als die von Luvimer™ 100 P. Dies ist für die Applikation als Spray oder Aerosol von Vorteil. Insgesamt wird ein Profil an Gebrauchs- und Anwendungseigenschaften gefunden, das Handelsprodukten überlegen ist.

Die Polymere P können aus Lösung zu Filmen ausgestrichen werden. Nach Trocknen zeigen diese Filme eine größere Härte als handelsübliche Haarfestigerpolymere. Das ist ebenfalls ein Hinweis auf ihre im Vergleich zu Handelsprodukten überlegene Eignung bei Verwendung als Haarfestiger.

Die Polymere P können auch erfindungsgemäß in Kosmetika eingesetzt werden. Auch bei der erfindungsgemäßen Verwendung in Kosmetika tritt beispielsweise der Vorteil der niedrigen Viskosität hervor.

Die Polymere P können allein aus Monomeren der Formel II oder in Mischung mit ethylenisch ungesättigten Verbindungen hergestellt werden. Bevorzugt sind 0,05 bis 100 Gew.-% der eingesetzten Monomeren Monomere der Formel II und 0 bis 99,95 Gew.-% weitere copolymerisierbar Monomere.

Alternativ besteht die Möglichkeit, die Polymere P aus Präpolymeren zu erhalten. So können beispielsweise Präpolymere, die aus Acrylsäure, Methacrylsäure und den Estern, Amiden, Halogeniden und sonstigen Derivaten dieser Säuren hergestellt wurden, zu Polymeren umgesetzt werden, die eine Gruppe der Formel I enthalten. Insbesondere können so mit 2-Hydroxyethyl-(ethylenharnstoff) (CAS Nr. 3699-54-5) Polymere gewonnen werden, die Wiederholungseinheiten vom Typ III enthalten. Die auf diese Weise aus Präpolymeren synthetisierten Polymere P können ebenfalls erfindungsgemäß zur Verwendung in kosmetischen und haarkosmetischen Präparaten eingesetzt werden.

In bevorzugten Ausführungen der Erfindung werden Polymere P für den erfindungsgemäßen Anwendungszweck eingesetzt, die durch Copolymerisation aus Monomeren der Formel II mit geeigneten copolymerisierbaren Monomeren (E) erhalten werden, so daß die Polymeren zusätzlich Wiederholungseinheiten z.B. der Formeln IV, V, VI, VII und/oder VIII enthalten, 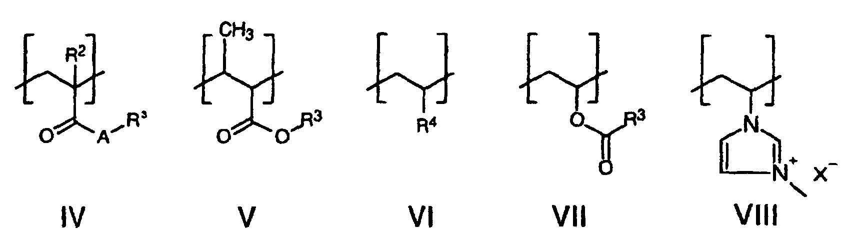 wobei
- R²: für H oder Methyl,
- R³: für H oder verzweigte oder unverzweigte Alkylreste, die mit einen Hydroxy- oder Amingruppe substituiert sein können,
- R⁴: für 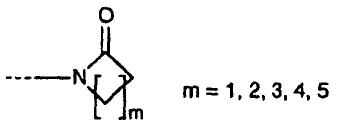
- A: für O oder NH,
- X⁻: für ein Anion, insbesondere Cl⁻, SO₄CH₃⁻, SO₄C₂H₅⁻, H₂PO₄⁻ steht.
aufweisen können.

Als Monomer der Formel II ist Ureidomethacrylat (R¹ = CH₃, R = H) besonders bevorzugt.

Als geeignete copolymerisierbare Monomere (E) können bevorzugt ethylenisch ungesättigte Monomere verwendet werden. Dabei kann entweder ein einzelnes Monomer oder Kombinationen von zwei oder mehr Monomeren verwendet werden. Mit copolymerisierbar ist gemeint, daß die verwendeten Monomere unter Verwendung irgendeiner konventionellen synthetischen Methode polymerisiert werden können.
Beispielsweise können dies Lösungspolymerisation, Emulsionspolymerisation, umgekehrte Emulsionspolymerisation, Suspensionspolymerisation, umgekehrte Suspensionspolymerisation oder Fällungspolymerisation sein, ohne daß die verwendbaren Methoden darauf beschränkt sind. Bei der Lösungspolymerisation kann Wasser oder übliche organische Lösungsmittel als Lösungsmittel verwendet werden. Auch die Herstellung in der Schmelze ist möglich.

Monomere, die mit einer durch freie Radikale initiierten Reaktion polymerisiert werden können, sind bevorzugt. Der Begriff ethylenisch ungesättigt bedeutet, daß die Monomere zumindest eine polymerisierbare Kohlenstoff-Kohlenstoff-Doppelbindung besitzen, die mono-, di-, tri-, oder tetrasubstituiert sein kann.

Die Monomere (E) können im Polymer P bis 99,95 Gew.-%, bevorzugt 85 bis 98 Gew.-%, ausmachen.

Die bevorzugten ethylenisch ungesättigten Monomere (E) können durch die folgende allgemeine Formel beschrieben werden:

X-C(O)CR⁷=CHR⁶

wobei
X ausgewählt ist aus der Gruppe der Reste -OH, -OM, -OR⁸, NH₂, -NHR⁸, N(R⁸)₂;
M ist ein Kation, ausgewählt aus der Gruppe, bestehend aus Na+, K+, Mg++, Ca++, Zn++, NH4+, Alkylammonium, Dialkylammonium, Trialkylammonium und Tetraalkylammonium;
die Reste R⁸ können identisch oder verschieden ausgewählt werden aus der Gruppe, bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl.
R⁷ und R⁶ sind unabhängig voneinander, ausgewählt aus der Gruppe, bestehend aus -H, C₁-C₈ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren (E) sind zum Beispiel Acrylsäure und deren Salze, Ester und Amide. Die Salze können von jedem beliebigen nicht toxischen Metall, Ammonium oder substituierten Ammonium-Gegenionen abgeleitet sein.

Die Ester können abgeleitet sein von C₁-C₄₀ linearen, C₃-C₄₀ .verzweigtkettigen, oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin, and 1,2,6-Hexantriol, von Aminoalkoholen oder von Alkoholethern wie Methoxyethanol und Ethoxyethanol oder Polyethylenglykolen.

Ferner eignen sich N,N-Dialkylaminoalkylacrylate- und methacrylate und N-Dialkylaminoalkylacryl- und -methacrylamide der allgemeinen Formel (EII) 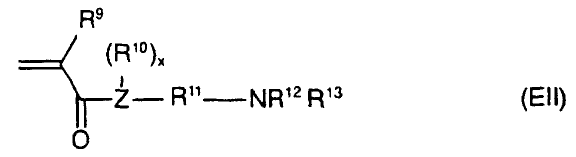 mit
- R⁹ =: H, Alkyl mit 1 bis 8 C-Atomen,
- R¹⁰ =: H, Methyl,
- R¹¹ =: Alkylen mit 1 bis 24 C-Atomen, optional substituiert durch C₁-C₆-Alkyl,
- R¹², R¹³ =: C₁-C₄₀ Alkylrest,
- Z =: Stickstoff für x = 1 oder Sauerstoff für x = 0.

Die Amide können unsubstituiert, N-Alkyl oder N-alkylamino monosubstituiert, oder N,N-dialkylsubstituiert oder N,N-dialkylamino disubstituiert sein, worin die Alkyl- oder Alkylaminogruppen von C₁-C₄₀ linearen, C₃-C₄₀ verzweigtkettigen, oder C₃-C₄₀ carbocyclischen Einheiten abgeleitet sind. Zusätzlich können die Alkylaminogruppen quaternisiert werden.

Bevorzugte Monomere der Formel EII sind N,N-Dimethylaminomethyl(meth)acrylat, N,N-Diethylaminomethyl(meth)acrylat, N,N-Dimethylaminoethyl(meth)acrylat, N,N-Diethylaminoethyl(meth)acrylat.

Ebenfalls verwendbare Monomere (E) sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe, bestehend aus C₁-C₄ Alkyl, -CN, COOH, besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Monomere (E) sind Vinyl- und Allylester von C₁-C₄₀ linearen, C₃-C₄₀ verzweigtkettigen oder C₃-C₄₀ carbocyclische Carbonsäuren (z.B. Vinylacetat, Vinylpropionat, Vinylneononanoat, Vinylneoundekansäure oder t-Butyl-benzoesäure-vinylester); Vinyl- oder Allylhalogenide, bevorzugt Vinylchlorid und Allylchlorid, Vinylether, bevorzugt Methyl-, Ethyl-, Butyl-, oder Dodecylvinylether, Vinylformamid, Vinylmethylacetamid, Vinylamin; Vinyllactame, bevorzugt Vinylpyrrolidon und Vinylcaprolactam, Vinyl- oder Allyl-substituierte heterocyclische Verbindungen, bevorzugt Vinylpyridin, Vinyloxazolin und Allylpyridin.

Weiterhin sind N-Vinylimidazole der allgemeinen Formel EIII geeignet, worin R¹⁴ bis R¹⁶ unabhängig voneinander für Wasserstoff, C₁-C₄-Alkyl oder Phenyl steht:

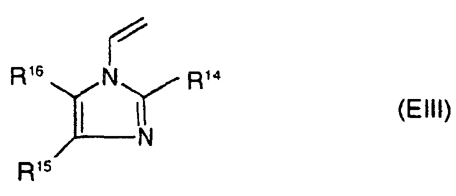

Weitere geeignete Monomere (E) sind Diallylamine der allgemeinen Formel (EIV) 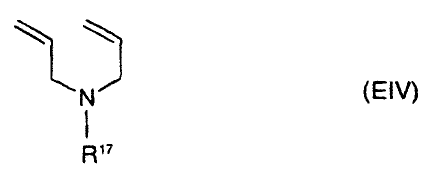 mit R¹⁷= C₁- bis C₂₄-Alkyl.

Weitere geeignete Monomere (E) sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere.

Besonders geeignete Monomere (E) sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butyl-ethacrylat, 2-Ethylhexylethacrylat, Decylethacrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren, wie z.B. Acrylamidopropansulfonsäure;
Acrylamid, Methacrylamid, Ethacrylamid, N-Methylacrylamid, N,N-Dimethylacrylamid, N-Ethylacrylamid, N-Isopropylacrylamid, N-Butylacrylamid, N-t-Butylacrylamid, N-Octylacrylamid, N-t-Octylacrylamid, N-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylimidazol, N,N-Dimethylaminomethyl(meth)acrylat, N,N-Diethylaminomethyl(meth)acrylat, N,N-Dimethylaminoethyl(meth)acrylat, N,N-Diethylaminoethyl(meth)acrylat, N,N-Dimethylaminobutyl(meth)acrylat, N,N-Diethylaminobutyl(meth)acrylat, N,N-Dimethylaminohexyl(meth)acrylat, N,N-Dimethylaminooctyl(meth)acrylat, N,N-Dimethylaminododecyl(meth)acrylat, N-[3-(dimethylamino)propyl]methacrylamid, N-[3-(dimethylamino)propyl]acrylamid, N-[3-(dimethylamino)butyl]methacrylamid, N-[8-(dimethylamino)octyl]methacrylamid, N-[12-(dimethylaminoldodecyl]methacrylamid, N-[3-(diethylamino)propyl]methacrylamid, N-[3-(diethylamino)propyl]acrylamid;
Maleinsäure, Fumarsäure, Maleinsäureanhydrid und seine Halbester, Crotonsäure, Itaconsäure, Diallyldimethylammoniumchlorid, Vinylether (z.B. Methyl-, Ethyl-, Butyl-, oder Dodecylvinylether), Vinylformamid, Vinylmethylacetamid, Vinylamin; Methylvinylketon, Maleimid, Vinylpyridin, Vinylimidazol, Vinylfuran, Styrol, Styrolsulfonat, Allylalkohol, und Mischungen daraus.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, N-t-Butylacrylamid, N-Octylacrylamid, 2-Hydroxyethylacrylat, Hydroxypropylacrylat, 2-Hydroxyethylmethacrylat, Hydroxypropylmethacrylat, Alkylenglykol(meth)acrylate, ungesättigte Sulfonsäuren wie zum Beispiel Acrylamidopropansulfonsäure, Vinylpyrrolidon, Vinylcaprolactam, Vinylether (z.B. Methyl-, Ethyl-, Butyl-, oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N-[3-(dimethylamino)propyl]methacrylamid ; 3-Methyl-1-vinylimidazoliumchlorid, 3-Methyl-1-vinylimidazoliummethylsulfat, N,N-Dimethylaminoethylmethacrylat, N-[3-(dimethylamino)propyl]methacrylamid quaternisiert mit Methylchlorid, Methylsulfat oder Diethylsulfat.

Monomere mit einem basischen Stickstoffatom können dabei auf folgende Weise quaternisiert werden:

Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der basischen Amine kann auch mit Alkylenoxiden wie Ethylenoxid oder Propylenoxid in Gegenwart von Säuren durchgeführt werden. Bevorzugte Quaternierungsmittel sind Methylchlorid, Dimethylsulfat oder Diethylsulfat.

Die Quaternisierung kann vor der Polymerisation oder nach der Polymerisation durchgeführt werden.

Außerdem können die Umsetzungsprodukte von ungesättigten Säuren, wie z.B. Acrylsäure oder Methacrylsäure, mit einem quaternisierten Epichlorhydrin der allgemeinen Formel (EV) eingesetzt werden (R¹⁸ = C₁- bis C₄₀ Alkyl)

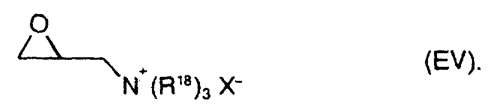

Beispiele hierfür sind z.B. (Meth)acryloyloxyhydroxypropyltrimethylammoniumchlorid und (Meth)acryloyloxyhydroxypropyltriethylammoniumchlorid.

Die basischen Monomere können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Zusätzlich zu den oben genannten Monomeren können als Monomere (E) sogenannte Makromonomere wie zum Beispiel silikonhaltige Makromonomere mit ein oder mehreren radikalisch polymerisierbaren Gruppen oder Alkyloxazolinmakromonomere eingesetzt werden, wie sie z.B. in der EP 408 311 beschrieben sind.

Des weiteren können fluorhaltige Monomere, wie sie beispielsweise in der EP 558 423 beschrieben sind, vernetzend wirkende oder das Molekulargewicht regelnde Verbindungen in Kombination oder alleine eingesetzt werden.

Die Erfindung betrifft außerdem die Verwendung von Polymeren, die unter Einsatz von Monomeren der Formel II hergestellt werden oder wenigstens eine Wiederholungseinheit der Formel III enthalten, dadurch gekennzeichnet, daß sie keine Gruppen enthalten, die Tensidcharakter verbunden mit einer Acetoacetoxy-Funktion aufweisen, in Kosmetika, insbesondere in haarkosmetischen Erzeugnissen, Haarpflegemitteln und Haarfestigern, sowie die Verwendung von Polymeren, die unter Einsatz von Monomeren der Formel II hergestellt werden oder wenigstens eine Wiederholungseinheit der Formel III enthalten, dadurch gekennzeichnet, daß das Polymer in Lösung oder durch Substanzpolymerisation hergestellt wird oder im gelösten Zustand verwendet wird, in Kosmetika, insbesondere in haarkosmetischen Erzeugnissen, Haarpflegemitteln und Haarfestigern.

Die Erfindung betrifft auch Haarfestiger, enthaltend ein Polymer, an das Gruppen der Formel I mit den oben angegebenen Bedeutungen gebunden sind.

Der Begriff "Haarfestiger" umfaßt dabei bevorzugt Haarspray, Schaumfestiger, Haarmousse, Haargel, Festigerlotion und Festiger-Creme.

Das Polymer P kann bei der erfindungsgemäßen Verwendung in Lösung, Suspension, Emulsion oder Schmelze hergestellt werden oder im gelösten, suspendierten oder emulgierten Zustand zum Einsatz gebracht werden.

Das Polymer P kann insbesondere in einem Fluid gelöst, suspendiert oder emulgiert werden, das wenigstens einen der Stoffe Dimethylether, Propan, Butan, Ethanol, Isopropanol, Wasser und/oder halogenierte Kohlenwasserstoffe enthält.

Die Erfindung betrifft weiterhin die Verwendung von Polymeren, an die Gruppen der Formel I gebunden sind, und die zu 95 bis 99,95 Gew.-% aus einem Monomeren bestehen, das keine Gruppen der Formel I aufweist, in Kosmetika, insbesondere haarkosmetischen Erzeugnissen.

Außerdem betrifft die Erfindung Copolymere, die aus
a) 0,05 bis 90 Gew.-% Monomeren der Formel IIa 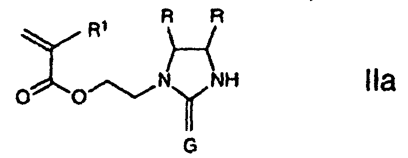 worin
   - R¹: H oder CH₃,
   - G: 0 oder NH und
   - R: H oder beide R die Gruppe -NH-C(O)-NH-
   bedeuten, und
b) 99,95 bis 10 Gew.-% Vinylcaprolactam und/oder Vinylpyrrolidon bestehen.

Als Regler für die Polymerisation der Polymere P können die üblichen dem Fachmann bekannten Verbindungen, wie z.B. Schwefelverbindungen (z.B. Mercaptoethanol, 2-Ethylhexylthioglykolat, Thioglykolsäure oder Dodecylmercaptan), sowie Tribromchlormethan oder andere Verbindungen, die regelnd auf das Molekulargewicht der erhaltenen Polymerisate wirken, verwendet werden.

Es können gegebenenfalls auch thiolgruppenhaltige Silikonverbindungen eingesetzt werden.

Bevorzugt werden silikonfreie Regler eingesetzt.

Als vernetzende Monomere können Verbindungen mit mindestens zwei ethylenisch ungesättigten Doppelbindungen eingesetzt werden, wie z.B. Ester von ethylenisch ungesättigten Carbonsäuren, wie Acrylsäure oder Methacrylsäure und mehrwertigen Alkoholen, Ether von mindestens zweiwertigen Alkoholen, wie z.B. Vinylether oder Allylether. Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen. Ferner geeignet sind Amide der Acryl- und Methacrylsäure und N-Allylamine von mindestens zweiwertigen Aminen, wie z.B. 1,2-Diaminoethan, 1,3-Diaminopropan. Ferner sind Triallylamin oder entsprechende Ammoniumsalze, N-Vinylverbindungen von Harnstoffderivaten, mindestens zweiwertigen Amiden, Cyanuraten oder Urethanen geeignet. Weitere geeignete Vernetzer sind Divinyldioxan, Tetraallylsilan oder Tetravinylsilan.

Besonders bevorzugte Vernetzer sind beispielsweise Methylenbisacrylamid, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen. die mit Ethylenoxid und/oder Propylenoxid und/oder Epichlorhydrin umgesetzt worden sind.

Bei der Polymerisation der Monomeren (E) oder bei der erfindungsgemäßen Verwendung der Polymere P können gegebenenfalls auch andere Polymere, wie z.B. Polyamide, Polyurethane, Polyester, Homo- und Copolymere von ethylenisch ungesättigten Monomeren zugegen sein. Beispiele für solche zum Teil auch in der Kosmetik eingesetzten Polymeren sind die unter den Handelsnamen bekannten Polymere Amerhold™, Ultrahold™, Ultrahold Strong™, Luviflex™ VBM, Luvimer™, Acronal™, Acudyne™, Stepanhold™, Lovocryl™, Versatyl™, Amphomer™ oder Eastman AQ™.

Die Monomere (E) können, sofern sie ionisierbare Gruppen enthalten, vor oder nach der Polymerisation, zum Teil oder vollständig mit Säuren oder Basen neutralisiert werden, um so z.B. die Wasserlöslichkeit oder -dispergierbarkeit auf ein gewünschtes Maß einzustellen.

Als Neutralisationsmittel für Säuregruppen tragende Monomere können z.B. Mineralbasen wie Natriumcarbonat, Alkalihydroxide sowie Ammoniak, organische Basen wie Aminoalkohole, speziell 2-Amino-2-Methyl-1-Propanol, Monoethanolamin, Diethanolamin, Triethanolamin, Triisopropanolamin, Tri[(2-hydroxy)1-propyl]amin, 2-Amino-2-Methyl-1,3-Propandiol, 2-Amino-2-hydroxymethyl-1,3-Propandiol, sowie Diamine, wie z.B. Lysin, verwendet werden.

Als Neutralisationsmittel für kationisierbare Gruppen tragende Monomere können z.B. Mineralsäuren wie Salzsäure, Schwefelsäure oder Phosphorsäure sowie organische Säuren wie Carbonsäuren, Milchsäure, Zitronensäure oder andere eingesetzt werden.

Es können weiterhin Hilfsstoffe wie Weichmacher, Filmbildehilfsmittel, Pigmente, Parfums oder andere, alleine oder in Kombination, bei der Polymerisation anwesend sein und/oder nach der Polymerisation zugefügt werden.

In einer bevorzugten Ausführung der Erfindung werden zu Herstellung der Polymere P keine ethylenisch ungesättigten Monomere, die Tensidcharakter verbunden mit einer Acetoacetoxy-Funktion aufweisen, verwendet (WO 97/45468). Dadurch ist es möglich, dem Polymer erst nach Herstellung durch Mischen mit geeigneten Tensiden die für den jeweiligen Anwendungszweck optimalen Grenzflächeneigenschaften zu geben.

Bei der erfindungsgemäßen Verwendung der Polymere P als Haarfestiger ist es vorteilhaft, die Glastemperatur der Polymerisate durch geeignete Kombination von ethylenisch ungesättigten Monomeren auf Werte größer 20°C einzustellen.

Besonders bevorzugte ethylenisch ungesättigte Monomere (E) für den Einsatz in Haarfestigerpolymeren sind n-Butylacrylat, Methylmethacrylat, t-Butylacrylat, Ethylacrylat, Styrol, N-t-Butylacrylamid, Methacrylsäure, Acrylsäure, Crotonsäure, Vinylacetat, Vinylpropionat, Vinylcaprolactam, Vinylpyrrolidon, Vinylimidazol und N-Methylvinylimidazoliniumsalz.

Erfindungsgemäß ist ferner die Verwendung von Polymeren P in Mischung mit anderen Polymeren. Besonders geeignet sind hier Polymere, die üblicherweise in der Kosmetik eingesetzt werden. Derartige Polymere sind beispielsweise Poly(meth)acrylate, Polyester, Polyurethane, Polymere mit quaternierten Stickstoffatomen, Polymere mit Vinylpyrrolidon-, Vinylcaprolactam-, Vinyalacetat- oder Vinylether-Wiederholungseinheit, Polymere mit N-alkylierten Acrylamid-Wiederholungseinheiten, Polymere mit 1-(N,N-Dimethylamino)-2-ethylmethacrylat-Wiederholungseinheiten, Polymere mit Imidazolyl-Resten (Handelsnamen beispielsweise Amerhold™, Ultrahold™, Ultrahold Strong™, Luviflex™ VBM, Luvimer™, Acronal™, Acudyne™, Stepanhold™, Lovocryl™, Versatyl™, Amphomer™ oder Eastman AQ™).

Erfindungsgemäß ist außerdem die Verwendung von Polymeren, die neben den genannten Monomeren andere Gruppierungen (IX) enthalten, die beispielsweise aus den in DE 4301673 A1 genannten Nebenprodukten der Synthese von Ureidomethacrylat hervorgehen, insbesondere Hydroxyethylethylenharnstoff (CAS Nr. 3699-54-5) und N-(Methacryloyloxyethyl)-N'-(methacryloyl)ethylenharnstoff (CAS Nr. 157312-17-9).

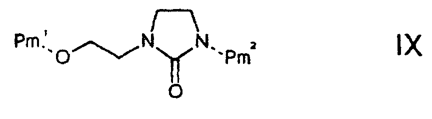

Wenigstens einer der beiden Reste Pm¹ und Pm² steht für einen hochmolekularen Rest.

### Beispiele

### Beispiel 1 bis 4

Zu einer gerührten Vorlage werden 50 g Zulauf 1 und 3,75 g Zulauf 2 getropft. Die Mischung wird dann auf 78°C erhitzt. Danach werden innerhalb 1,5 h der Rest von Zulauf 1 und innerhalb von 2 h der Rest von Zulauf 2 zugetropft. Die Mischung wird weitere 2 h gerührt. Danach wird Zulauf 3 innerhalb von 15 min. zugetropft und noch 2 h bei 78°C gerührt.

### Beispiel 1

| | |
|---|---|
| Vorlage | 175 g Ethanol |
| Zulauf 1 | 248 g t-Butylacrylat, 82,5 g Methacrylsäure, 37,5 g Ethylacrylat, 15,5 g einer Lösung von 50 Gew.-% Methacrylsäure-[2- (2-oxoimidazolidin-1-yl)-ethylester] (CAS Nummer 86261-90-7) in Wasser |
| Zulauf 2 | 1,5 g t-Butyl-perpivalat, 100 g Ethanol |
| Zulauf 3 | 0,5 g t-Butyl-perpivalat, 57,5 g Ethanol |

### Beispiel 2

| | |
|---|---|
| Vorlage | 175 g Ethanol |
| Zulauf 1 | 248 g t-Butylacrylat, 82,5 g Methacrylsäure, 37,5 g Ethylacrylat, 75 g Ethanol 36,6 g einer Lösung von 50 Gew.-% Methacrylsäure-[2- (2-oxoimidazolidin-1-yl)-ethylester] (CAS Nummer 86261-90-7) in Wasser |
| Zulauf 2 | 1,5 g t-Butyl-perpivalat, 100 g Ethanol |
| Zulauf 3 | 0,5 g t-Butyl-perpivalat, 57.5 g Ethanol |

### Beispiel 3

| | |
|---|---|
| Vorlage | 200 g Ethanol |
| Zulauf 1 | 248 g t-Butylacrylat, 82,5 g Methacrylsäure, 37,5 g Ethylacrylat 18,8 g einer Lösung von 50 Gew.-% Methacrylsäure-[2-(2-oxoimidazolidin-1-yl)-ethylester] (CAS Nummer 86261-90-7) in Wasser |
| Zulauf 2 | 2,5 g t-Butyl-perpivalat, 100 g Ethanol |
| Zulauf 3 | 0,5 g t-Butyl-perpivalat, 57,5 g Ethanol |

### Beispiel 4

| | |
|---|---|
| Vorlage | 200 g Ethanol |
| Zulauf 1 | 248 g t-Butylacrylat, 82,5 g Methacrylsäure, 37,5 g Ethylacrylat 75,1 g einer Lösung von 25 Gew.-% Methacrylsäure-[2-(2-oxoimidazolidin-1-yl)-ethylester] (CAS Nummer 86261-90-7) in Methylmethacrylat |
| Zulauf 2 | 2,5 g t-Butyl-perpivalat, 75 g Ethanol |
| Zulauf 3 | 0,5 g t-Butyl-perpivalat, 57,5 g Ethanol |

### Beispiel 5

### Bestimmung der Pendelhärte nach König und Redispergierbarkeit in Wasser

Die Polymere aus den Beispielen 1 bis 4 und ein handelsübliches Polymer werden jeweils in Ethanol gelöst und mit 2-Amino-2-Methyl-1-Propanol (AMP) neutralisiert, so daß der Feststoffgehalt bei 20 Gew.-% liegt. Die Lösungen werden mit einer Kastenrakel (Schlitzweite 200 µm) auf einer Glasplatte zu Filmen ausgerakelt und 24 h bei Raumtemperatur getrocknet. Die Pendelhärte nach König und die Redispergierbarkeit in Wasser ist aus der Tabelle zu entnehmen.

Zu Vergleichszwecken wird das handelsübliche Haarfestigerpolymer Luvimer™ 100 P der Firma BASF Aktiengesellschaft verwendet. Luvimer 100 P ist ein Copolymer aus tert.-Butylacrylat, Ethylacrylat und Methacrylsäure.

**Tabelle**

| Polymer | Pendelhärte | Redispergierbarkeit |
|---|---|---|
| Beispiel 1 | 162 | vollständig |
| Beispiel 2 | 153 | vollständig |
| Beispiel 3 | 148 | vollständig |
| Beispiel 4 | 155 | vollständig |
| Luvimer™ 100 P | 143 | vollständig |

### Beispiel 6

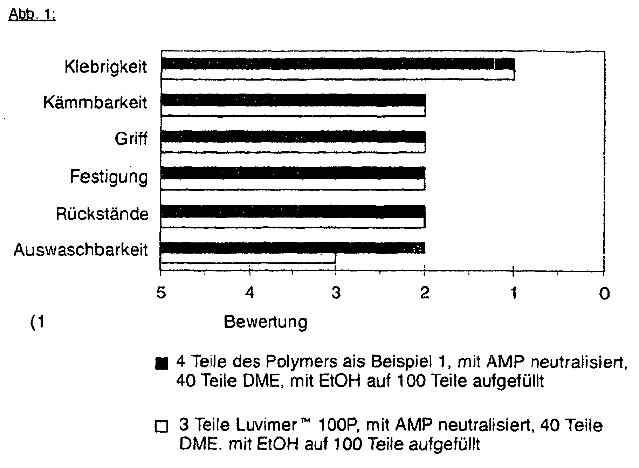

Prüfungen des Polymers aus Beispiel 1 und eines handelsüblichen Polymers (Luvimer™ 100 P von der Fa. BASF Aktiengesellschaft) auf ihre Eignung als Haarbehandlungsmittel: Zur Prüfung werden Kunstköpfe mit menschlichem Haar im Halbseitentest mit einer definierten Menge aus einer Standardformulierung der Polymere (3 bis 4 Gew.-Teile des Polymeren, Neutralisation des Polymeren mit AMP, 40 Gew.-Teile Dimethylether, Auffüllen auf 100 Gew.-Teile mit Ethanol) eingesprüht. Nach dem Trocknen werden die Eigenschaften des Haars (Klebrigkeit, Kämmbarkeit, Griff, Festigung, Rückstände, Auswaschbarkeit) sensorisch beurteilt. Es zeigt sich, daß das Eigenschaftsprofil des Polymers aus Beispiel 1 dem Handelsprodukt Luvimer™ 100 P überlegen ist (Abbildung 1).

### Beispiel 7

Die Auswaschbarkeit wird wie in Beispiel 2 verglichen, jedoch werden die Polymere in VOC-80-Formulierungen eingesetzt (4 Gew.-Teile des Polymeren, Neutralisation des Polymeren mit 2-Aminomethyl-1-propanol, 16 Gew.-Teile Wasser, 40 Gew.-Teile Dimethylether, Auffüllen auf 100 Gew.-Teile mit Ethanol). Die Auswaschbarkeit für das Polymer aus Beispiel 1 wird als gut (Bewertung "2") beurteilt, während Luvimer 100 P als befriedigend (Bewertung "3") beurteilt wird.

### Beispiel 8

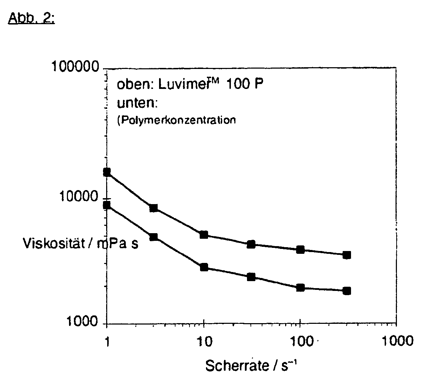

Lösungen des Polymers aus Beispiel 1 und von Luvimer™ 100 P, beide 45 Gew.-% in Ethanol, werden mit einem Haake Rheometer untersucht. Als Meßeinrichtung werden koaxiale Zylinder verwendet. Das Ergebnis zeigt, daß die Viskosität des Polymers aus Beispiel 1 niedriger ist als die von Luvimer™ 100 P (Abbildung 2).

## Patentansprüche

1. Verwendung von Polymeren, an die Gruppen der Formel I gebunden sind, 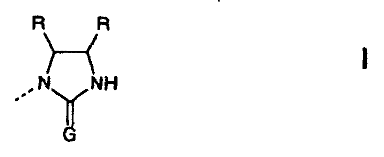 wobei
R für H steht oder beide R für die Gruppe -NH-C(O)-NH- und
G für O oder NH steht,
als Haarfestigerpolymere.

2. Verwendung von Polymeren nach Anspruch 1, **dadurch gekennzeichnet, daß** das Polymer unter Einsatz von Monomeren der Formel II hergestellt wird oder Wiederholungseinheiten der Formel III enthält, 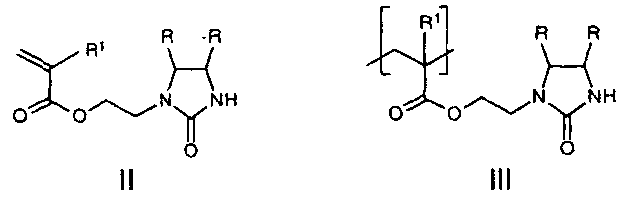 wobei
R¹ H oder CH₃ bedeutet und
R die in Anspruch 1 angegebene Bedeutung hat.

3. Verwendung von Polymeren nach Anspruch 1, **dadurch gekennzeichnet, daß** das Polymer durch Polymerisation aus Monomeren mit olefinischen Doppelbindungen hergestellt wird, und daß 0,05 bis 100 Gew.-% des Monomers ein Monomer der Formel II und 0 bis 99,95 Gew.-% ein weiteres copolysierbares Monomer E sind.

4. Verwendung von Polymeren nach Anspruch 1, **dadurch gekennzeichnet, daß** das Polymer Wiederholungseinheiten der Formel III und wenigstens eine der Wiederholungseinheiten der Formeln IV, V, VI, VII und VIII enthält, 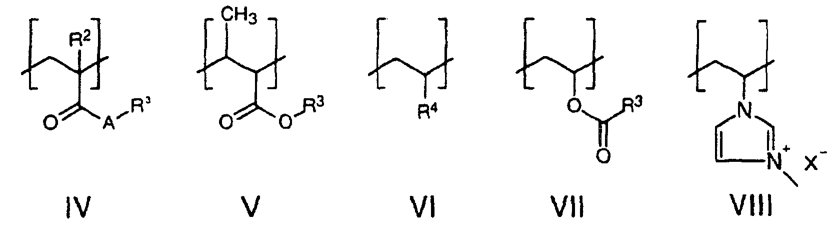 wobei
R² für H oder Methyl,
R³ für H oder verzweigte oder unverzweigte Alkylreste, die mit einer Hydroxy- oder Amingruppe substituiert sein können,
R⁴ für 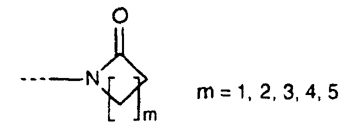
A für O oder NH,
X⁻ für ein Anion, insbesondere Cl⁻, SO₄CH₃⁻, SO₄C₂H₅⁻, H₂PO₄⁻ steht.

5. Verwendung von Polymeren nach Anspruch 1, **dadurch gekennzeichnet, daß** das Polymer in Lösung, in Suspension, in Emulsion oder in der Schmelze hergestellt.wird, oder daß das Polymer im gelösten, suspendierten oder emulgierten Zustand zum Einsatz gebracht wird.

6. Verwendung von Polymeren nach Anspruch 1, **dadurch gekennzeichnet, daß** das Polymer in einem Fluid gelöst, suspendiert oder emulgiert ist, das wenigstens einen der Stoffe Dimethylether, Propan, Butan, Ethanol, Isopropanol, Wasser und/oder halogenierte Kohlenwasserstoffe enthält.

7. Verwendung von Polymeren, die unter Einsatz eines Monomeren der Formel II hergestellt werden oder wenigstens eine Wiederholungseinheit der Formel III enthalten, **dadurch gekennzeichnet, daß** sie keine Gruppen enthalten, die Tensidcharakter verbunden mit einer Acetoacetoxy-Funktion aufweisen, in Kosmetika, insbesondere in haarkosmetischen Erzeugnissen, Haarpflegemitteln und Haarfestigern.

8. Verwendung von Polymeren nach Anspruch 7, die unter Einsatz von Monomeren der Formel II hergestellt werden oder wenigstens eine Wiederholungseinheit der Formel III enthalten, und zu wenigstens 75 Gew.-% aus Monomeren bestehen, die aus der Gruppe n-Butylacrylat, Methylmethacrylat, t-Butylacrylat, Ethylacrylat, Styrol, N-t-Butylacrylamid, Methacrylsäure, Acrylsäure, Crotonsäure, Vinylacetat, Vinylpropionat, Vinylcaprolactam, Vinylpyrrolidon, Vinylimidazol und N-Methylvinylimidazoliniumsalz entnommen sind, in Kosmetika, insbesondere in haarkosmetischen Erzeugnissen, Haarpflegemitteln und Haarfestigern.

9. Verwendung von Polymeren, die unter Einsatz von Monomeren der Formel II hergestellt werden oder wenigstens eine Wiederholungseinheit der Formel III enthalten, **dadurch gekennzeichnet, daß** das Polymer in Lösung oder durch Substanzpolymerisation hergestellt wird oder im gelösten Zustand verwendet wird, in Kosmetika, insbesondere in haarkosmetischen Erzeugnissen, Haarpflegemitteln und Haarfestigern.

10. Verwendung von Polymeren nach Anspruch 1, 7 oder 9 in Haarspray, Schaumfestiger, Haarmousse, Haargel, Festigerlotion oder Festiger-Creme.

11. Verwendung von Polymeren, an die Gruppen der Formel I, wie in Anspruch 1 definiert, gebunden sind, und die zu 95 bis 99,9 Gew.-% aus einem Monomeren bestehen, das keine Gruppen der Formel I aufweist, in Kosmetika, insbesondere haarkosmetischen Erzeugnissen.

12. Haarfestiger, enthaltend ein Polymer, an das Gruppen der Formel I gemäß Anspruch 1 gebunden sind.

13. Copolymere, die aus
a) 0,05 bis 90 Gew.-% Monomeren der Formel IIa 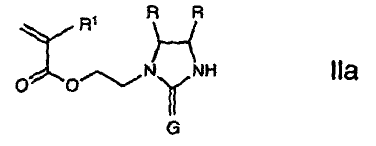 worin
R¹ H oder CH₃,
G O oder NH und
R H oder beide R die Gruppe -NH-C(O)-NH-
bedeuten, und
b) 99,95 bis 10 Gew.-% Vinylcaprolactam und/oder Vinylpyrrolidon
bestehen.

14. Verwendung von Polymeren nach Anspruch 1, 7, 9 oder 11, **dadurch gekennzeichnet, daß** die Polymeren in einer Mischung mit einem weiteren Polymer eingesetzt werden.

## Claims

1. The use of polymers to which groups of the formula I are bonded, 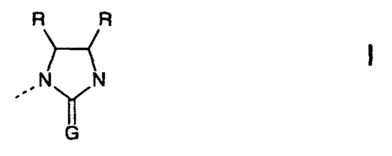 where
R is H or the two Rs are the group -NH-C(O)-NH-, and
G is O or NH,
as hair-setting polymers.

2. The use of polymers as claimed in claim 1, wherein the polymer is prepared using monomers of the formula II, or contains repeat units of the formula III, 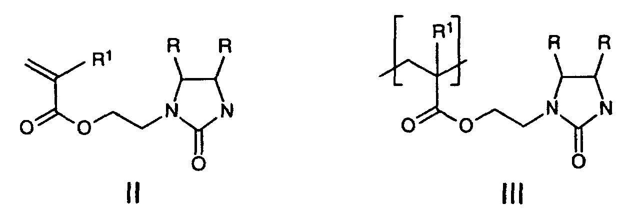 where
R¹ is H or CH₃, and
R is as defined in claim 1.

3. The use of polymers as claimed in claim 1, wherein the polymer is prepared by polymerization from monomers having olefinic double bonds, and wherein from 0.05 to 100% by weight of the monomer are a monomer of the formula II, and from 0 to 99.95% by weight are a further copolymerizable monomer E.

4. The use of polymers as claimed in claim 1, wherein the polymer contains repeat units of the formula III and at least one of the repeat units of the formulae IV, V, VI, VII and VIII, 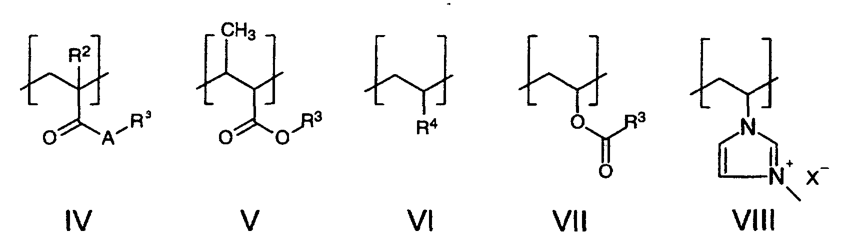 where
R² is H or methyl,
R³ is H or branched or unbranched alkyl radicals which may be substituted by a hydroxyl or amine group,
R⁴ is 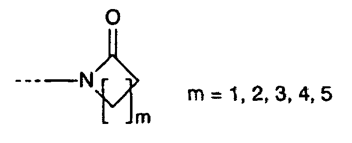
A is O or NH,
X⁻ is an anion, in particular Cl⁻, SO₄CH₃⁻, SO₄C₂H₅⁻, H₂PO₄⁻.

5. The use of polymers as claimed in claim 1, wherein the polymer is prepared in solution, in suspension, in emulsion or in the melt, or wherein the polymer is brought into the dissolved, suspended or emulsified state for use.

6. The use of polymers as claimed in claim 1, wherein the polymer is dissolved, suspended or emulsified in a fluid which comprises at least one of the substances dimethyl ether, propane, butane, ethanol, isopropanol, water and/or halogenated hydrocarbons.

7. The use of polymers which are prepared using a monomer of the formula II or contain at least one repeat unit of the formula III wherein the polymers do not contain any groups which have a surfactant character associated with an acetoacetoxy function, in cosmetics, in particular in hair cosmetic products, haircare compositions and hair-setting compositions.

8. The use of polymers as claimed in claim 7, which are prepared using monomers of the formula II or contain at least one repeat unit of the formula III, and at least 75% by weight of which consist of monomers from the group n-butyl acrylate, methyl methacrylate, t-butyl acrylate, ethyl acrylate, styrene, N-t-butylacrylamide, methacrylic acid, acrylic acid, crotonic acid, vinyl acetate, vinyl propionate, vinylcaprolactam, vinylpyrrolidone, vinylimidazole and N-methylvinylimidazolinium salt, in cosmetics, in particular in hair cosmetic products, haircare compositions and hair-setting compositions.

9. The use of polymers which are prepared using monomers of the formula II or contain at least one repeat unit of the formula III, wherein the polymer is prepared in solution or by bulk polymerization, or is used in the dissolved state, in cosmetics, in particular in hair cosmetic products, haircare compositions and hair-setting compositions.

10. The use of polymers as claimed in claim 1, 7 or 9 in hairspray, setting foam, hair mousse, hair gel, setting lotion or setting cream.

11. The use of polymers to which groups of the formula I, as defined in claim 1, are bonded, and from 95 to 99.9% by weight of which consist of a monomer which does not have groups of the formula I, in cosmetics, in particular hair cosmetic products.

12. A hair-setting composition comprising a polymer to which groups of the formula I as in claim 1 are bonded.

13. A copolymer which consists of
a) from 0.05 to 90% by weight of monomers of the formula IIa 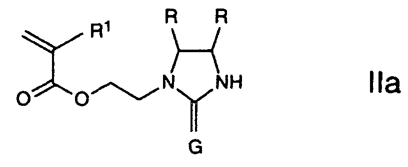 in which
R¹ is H or CH₃,
G is O or NH and
R is H or the two Rs are the group - NH-C(O)-NH-,
and
b) from 99.95 to 10% by weight of vinylcaprolactam and/or vinylpyrrolidone.

14. The use of polymers as claimed in claim 1, 7, 9 or 11, wherein the polymers are used in a mixture with a further polymer.

## Revendications

1. Utilisation de polymères auxquels sont liés des groupes de formule I 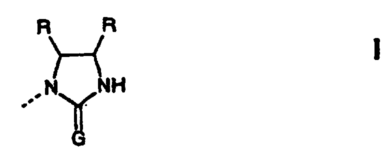 où
R désigne H ou les deux R représentent le groupe -NH-C(O)-NH- et
G désigne O ou NH,
comme polymères fixateurs capillaires.

2. Utilisation de polymères selon la revendications 1, **caractérisée par le fait que** le polymère est préparé par mise en oeuvre de monomères de formule II ou contient des unités récurrentes de formule III, 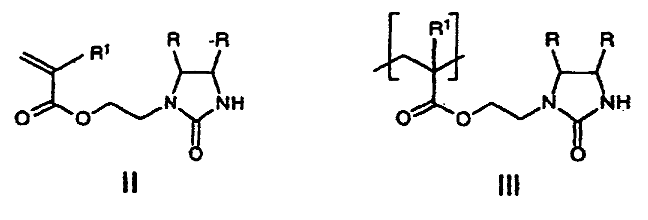 où
R¹ représente H ou CH₃ et
R a la signification indiquée dans la revendication 1.

3. Utilisation de polymères selon la revendication 1, **caractérisée par le fait que** le polymère est préparé par polymérisation de monomères ayant des doubles liaisons oléfiniques, et que 0,05 à 100 % en poids du monomère sont un monomère de formule II et 0 à 99,95 % en poids un autre monomère E copolymérisable.

4. Utilisation de polymères selon la revendication 1, **caractérisée par le fait que** le polymère contient des unités récurrentes de formule III et au moins l'une des unités récurrentes de formules IV, V, VI, VII et VIII, 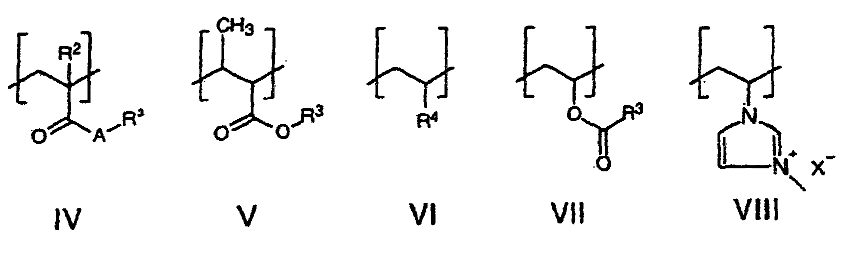 où
R² désigne H ou méthyle,
R³ représente H ou de restes alkyle ramifiés ou non-ramifiés, qui peuvent être substitués par un groupe hydroxy ou amine,
R⁴ représente 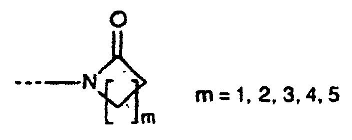
A désigne O ou NH,
X⁻ désigne un anion, en particulier Cl⁻, SO₄CH₃⁻, SO₄C₂H₅⁻, H₂PO₄⁻.

5. Utilisation de polymères selon la revendication 1, **caractérisée par le fait que** le polymère est préparé en solution, en suspension, en émulsion ou en masse fondue, ou que le polymère est mis en oeuvre à l'état dissous, en suspension ou en émulsion.

6. Utilisation de polymères selon la revendication 1, **caractérisée par le fait que** le polymère est dissous, mis en suspension ou émulsionné dans un fluide, qui contient au moins l'une des substances éther diméthylique, propane, butane, éthanol, isopropanol, eau et/ou hydrocarbure halogéné.

7. Utilisation de polymères qui sont préparés à partir d'un monomère de formule II ou contiennent au moins une unité récurrente de formule III, **caractérisée par le fait qu'**ils ne contiennent pas de groupes qui présentent un caractère tensioactif lié à une fonction acétoacétoxy, en cosmétique, en particulier dans des produits de cosmétique capillaire, des produits pour soins des cheveux et des renforçateurs pour cheveux.

8. Utilisation de polymères selon la revendication 7, qui sont préparés à partir de monomères de formule II ou contiennent au moins une unité récurrente de formule III, et consistent en au moins 75 % en poids en monomères qui sont choisis dans le groupe des acrylate de n-butyle, méthacrylate de méthyle, acrylate de tert-butyle, acrylate d'éthyle, styrène, N-tert-butylacrylamide, acide méthacrylique, acide acrylique, vinylpyrrolidone, vinylimidazole et sel de N-méthylvinyl-imidazolinium, en cosmétique, en particulier dans des produits de cosmétique capillaire, des produits pour soins des cheveux et des fixateurs capillaires.

9. Utilisation de polymères qui sont préparés à partir de monomères de formule II ou contiennent au moins une unité récurrente de formule III, **caractérisée par le fait que** le polymère est préparé en solution ou par polymérisation en substance ou est utilisé à l'état dissous, en cosmétique, en particulier dans des produits de cosmétique capillaire, des produits pour soins des cheveux et des fixateurs capillaires.

10. Utilisation de polymères selon la revendication 1, 7 ou 9 dans des sprays capillaires, des fixateurs en mousse, des mousses capillaires, des gels capillaires, des lotions fixatrices ou des crèmes fixatrices.

11. Utilisation de polymères auxquels sont liés des groupes de formule I, tels que définis dans la revendication 1, et qui consistent pour 95 à 99,9 % en poids en un monomère qui ne présente pas de groupes de formule I, en cosmétique, en particulier dans des produits de cosmétique capillaire.

12. Fixateur capillaire, contenant un polymère auquel sont liés des groupes de formule I selon la revendication 1.

13. Copolymères qui consistent en
a) 0,05 à 90 % en poids de monomères de formule IIa 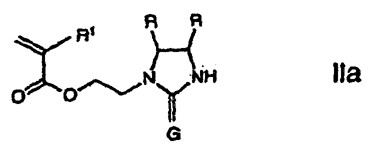 où
R¹ désigne H ou CH₃,
G représente O ou NH et
R désigne H ou les deux R désignent le groupe -NH-C(O)-NH-, et
b) 99,95 à 10 % en poids de vinylcaprolactame et/ou de vinylpyrrolidone.

14. Utilisation de polymères selon la revendication 1, 7, 9 ou 11, **caractérisée par le fait que** les polymères sont mis en oeuvre en un mélange avec un autre polymère.
